# EUROPEAN PATENT SPECIFICATION

(11) **EP 3 448 444 B1**
(45) Date of publication and mention of the grant of the patent: **12.10.2022**
(21) Application number: 17719573.2
(22) Date of filing: 25.04.2017
(51) Int. Cl.: A61L 27/12, A61L 27/24, A61L 27/36, A61L 27/56

(54) **VASCULARITY AFFINITY PRECURSOR STRUCTURE FOR MUSCULO-SKELETAL TISSUE HEALING**
GEFÄSSAFFINITÄTSVORLÄUFERSTRUKTUR FÜR MUSKEL-SKELETT-GEWEBEHEILUNG
STRUCTURE DE PRÉCURSEUR D'AFFINITÉ DE VASCULARITÉ POUR LA CICATRISATION DE TISSU MUSCULO-SQUELETTIQUE

(30) Priority: 25.04.2016 GB 201607127
(43) Date of publication of application: 06.03.2019
(73) Proprietor: Pharmaceutical Business Consultants Limited, Shannon, Co. Clare (IE)
(72) Inventor: GIANNOUDIS, Peter V, Leeds (GB); O'SULLIVAN, Carol, Castleconnell Limerick (IE); INSLEY, Gerard Michael, Rathkeale Limerick (IE); BURKE, Paul, Monaleen Limerick (IE); O'SULLIVAN, Regina, Mervue Galway (IE)
(74) Representative: HGF
(86) International application number: PCT/EP2017/059747
(87) International publication number: WO 2017/186692

(56) References cited:
- WO-A2-2008/103690
- WO-A2-2009/021209
- US-A1- 2008 033 572
- US-A1- 2013 345 826

## Description

### Field

The present invention relates to a medical device designed to treat Avascular Necrosis (AVN) and aid in musculo-skeletal tissue healing. In particular, the present invention relates to a device comprising a vascularity affinity precursor structure for musculo-skeletal tissue healing.

### Background Of The Invention

Injured bone, which may be necrotic and insufficiently vascularized, is a leading cause of bone pain and can clinically develop from a necrotic lesion to a full collapse of the bone, requiring total replacement of the anatomical region affected if early intervention does not occur or is unsuccessful. Whilst most common in the femoral head, and diagnosed as avascular necrosis (AVN), these injuries may affect and compromise other bony regions of the body such as the shoulder and ankle.

### AVASCULAR NECROSIS

Avascular necrosis (AVN) also known as Osteonecrosis (ON), Ischemic Bone necrosis, Bone Infarct and Aseptic Necrosis is a progressive bone degenerating disease, as a result of temporary or permanent loss of blood supply to the bones. Without access to the blood supply, the bone tissue dies and ultimately collapses. In time, this eventually leads to partial or complete collapse of the affected joint leading to pain and arthritis. AVN most commonly effects the hip joint but also occurs in the knee (Spontaneous osteonecrosis of the Knee, SPONK) and jaw (Bisphosphonate related osteonecrosis of the Jaw, BRONJ), and as previously stated in the shoulder and ankle. In the case where it is triggered by radiation it is known as Osteoradionecrosis (ORN) and when detected in children, the disease is termed Perthes disease. Currently, there are either non-surgical or surgical treatments which may try to slow the progression of the disease. Non-surgical treatments include medication, reduced weight bearing, range of motion exercise and electrical stimulation. These treatments can be used either alone or in combination. However, the pain relief gained from these treatments is only short term and ultimately the patient will require surgical intervention. The current surgical treatments available to treat AVN include Core decompression, Osteotomy, Bone Graft and Arthroplasty/total joint replacement. The majority of these treatments address the removal of dead bone only and are a means to relieve pain. These treatments however do not address disease progression, and they certainly do not provide bone healing and repair at the diseased site, nor even attempt to do so.

The present invention seeks to alleviate the disadvantages associated with known treatments for progressive bone degenerating disease including AVN.

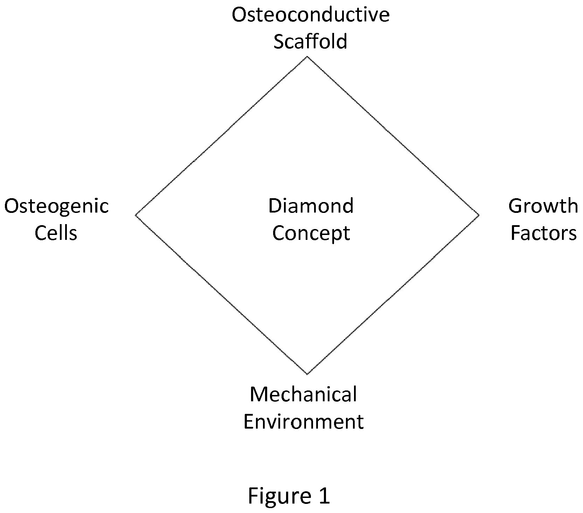

In the case of AVN and bone trauma or fracture, it is imperative to provide the body with an environment rich in angiogenic factors, (typically, through the provision of hematopoietic stem cells and endothelial progenitor cells). It has also been shown that it is important to provide the conditions proposed by Prof. V Giannoudis in the publication, Injury, 2007 Sep. 38-Suppl 4:S3-6. The conditions proposed by Prof. V Giannoudis, one of the co-inventors of the present invention, are referred to, collectively, as the 'Diamond Concept' and are shown schematically in Figure 1 of the publication, Injury, 2007 Sep. 38-Suppl 4:S3-6. The "Diamond Concept" as its name implies, provides a conceptual framework for the biological and mechanical prerequisite conditions required for the repair and regeneration of bone in injured states such as AVN or fracture. WO 2008/103690 describes a porous biocompatible matrix comprising porous calcium phosphate particles and a biocompatible binder, preferably collagen. A solution comprising PDGF is added onto the matrix. This solution can be e.g. platelet rich plasma. Further additional grafting materials can be added to the composition, e.g. autologous bone marrow.

The present invention provides a medical device configured to deliver each of the four key components conceptualised in the "Diamond Concept" which are necessary for repair and regeneration of bone following trauma.

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

The present invention provides a medical device comprising the following components:
1. Osteogenic Cells (found in Bone Marrow Aspirate (BMA),
2. Osteoconductive Scaffolds (provided by biological material, preferably, porous biological material, or provided by ceramic materials),
3. Growth Factors (present in and stimulated by both BMA and PRP) and a
4. Stable mechanical environment (optionally, provided by fixation device(s) or by a scaffold optionally, comprising a biomaterial scaffold).

The device of the present invention facilitates the promotion of a vascularized environment.

In one embodiment of the present invention, Bone Marrow Aspirate (BMA) (containing a suspension of autologous osteogenic and angiogenic cells) and/or Platelet-Rich Plasma (PRP) are used to facilitate revascularization, bone repair, regeneration and prevention of disease progression in conditions such as AVN.

The implantable device of the present invention has the advantage that it comprises all the requirements for bone repair and regeneration. By introducing all requirements in one implantable device, the rate and quality of vascularization, angiogenesis, bone repair and regeneration within the patient is improved in comparison to current treatments that only implement a portion of these.

Accordingly, the device of the present invention has the advantage that it provides an implantable device for musculo-skeletal tissue healing for bone repair and regeneration. The device of the present invention is capable of delivering, simultaneously, the four main components of the diamond concept, which are required for the repair and regeneration of bone in injured states such AVN or fracture, as well as facilitating a well vascularized host site all in one operative procedure.

In addition, where the device is implanted into a bone showing early stages of disease, the implantable device of the present invention aids in patient recovery and prevention of disease progression, thereby significantly reducing the potential need for more invasive surgical procedures with increased morbidity and mortality e.g. total hip arthroplasty.

There currently is no known device that is configured to deliver, to an injured bone site for the purposes of revascularisation and bone repair, all of the above referenced four requirements conceptualised in the "Diamond Concept".

### Summary

Accordingly, the present invention provides an implantable device comprising the technical features as claimed in claim 1.

Advantageous embodiments are provided in the dependent claims.

In one embodiment, the present invention relates to a device configured to provide a Vascularity Affinity Precursor Structure (VAPS) comprising the following components:
(a) A first component comprising a biological material configured to carry and maintain growth factors, (for instance, growth factors present in BMA and/or viable platelet rich plasma (PRP);
(b) A second component comprising a biological material configured to carry and maintain viable osteogenic and/or angiogenic cells; and
(c) A third component comprising a biomimetic bone generation phase, optionally comprising a calcium phase; and an osteoconductive scaffold which provides a stable mechanical environment.

Accordingly, in one embodiment, the present invention provides a device having a Vascularity Affinity Precursor Structure (VAPS) comprising the following components:
(a) A first component comprising a biological material configured to carry and maintain growth factors present in BMA and/or viable platelet rich plasma (PRP). The first component comprises a scaffold.
(b) A second component comprising a biological material configured to carry and maintain viable osteogenic and angiogenic cells. The second component preferably comprises osteogenic cells, provided by bone-marrow aspirate. The second component may also comprise angiogenic cells. The second component also comprises a scaffold.
(c) A third component comprising a biomimetic bone generation phase, comprising a calcium phase; and an osteoconductive scaffold which provides a stable mechanical environment. The osteoconductive scaffold of the present invention preferably comprises a biological material such as collagen; or a calcium based ceramic, preferably, a calcium phosphate compound. The biomimetic calcium phase preferably comprises a calcium phosphate compound. In one preferred embodiment, the calcium phosphate compound comprises Octacalcium Phosphate (OCP).

Advantageously, the biomimetic bone generation phase comprises a calcium phase comprises any one or more selected from the following group of calcium compounds: Octacalcium Phosphate (OCP) and/or Tri-Calcium Phosphate (TCP) and/or Hydroxyapatite (HA) and/or Dicalcium Phosphate Dihydrate (DCPD) and/or Dicalcium Phosphate Anhydrous (DCPA) and/or Amorphous Calcium Phosphate (ACP) and/or Calcium Carbonate.

In addition to cells and platelet-rich plasma (PRP), the device may also comprise pharmaceutical or medical devices e.g. Bone morphogenetic proteins (BMP's), antibiotics, antifungals, antivirals, bisphosphonates, growth factors, hormones, proteins and other inorganic minerals such as magnesium etc. Such pharmaceutical or medical devices may be added to, provided with or seeded with the device having a Vascularity Affinity Precursor Structure (VAPS), during manufacture or at point of care.

### DETAILED DESCRIPTION

The inventors have found that, in one embodiment of the present invention, by dispersing and/or encapsulating a calcium phosphate powder (such as octacalcium phosphate, a precursor in bone formation synthetic pathway) between two distinct biological scaffolds, each of which is designed to carry and maintain either angiogenic cells or osteogenic cells suspended in autologous bone marrow aspirate and PRP, the device of the present invention delivers all the required factors for vascularization, angiogenesis, bone repair and regeneration. In accordance with the present invention, this device providing a vascularity affinity precursor structure (VAPS) for promoting musculo-skeletal tissue healing, for the first time, allows surgeons to facilitate efficient vascularization and repair in a compromised bone (e.g. injured, avascular and necrotic bone). The structure and composition of the device also allows surgeons to personalise the device specific to the patients' needs at point of care for example the scaffold sponges may be loaded with pharmaceutical or medical devices e.g. bone morphogenetic proteins, antibiotics, antifungals, antivirals, bisphosphonates, growth factors, hormones, proteins and other inorganic minerals such as magnesium etc.

In a preferred embodiment, the device comprises a first scaffold component comprising collagen and a second scaffold component comprising collagen and a third component comprising OCP. The OCP may be provided dispersed between the first collagen scaffold component and the second collagen scaffold component; or alternatively, the OCP may be encapsulated within the first and/or second scaffold component(s).

### AUTOLOGUS CELLS - BONE MARROW ASPIRATE (BMA), PLATELET RICH PLASMA (PRP) & GROWTH FACTORS

In this invention, Bone Marrow Aspirate (BMA) containing a suspension of autologous osteogenic and angiogenic cells, or Platelet-Rich Plasma (PRP), or a combination of both, is used to facilitate revascularization, bone repair, regeneration and prevention of disease progression in conditions such as AVN.

The approach claimed herein for collection of the autologous angiogenic and osteogenic cells involves aspiration of the bone marrow aspirate (which also contains growth factors) from the iliac crest. Once isolated, the autologous angiogenic and osteogenic cells suspended in the bone marrow aspirate are added to the implant. Following implantation, the supply of cells (including MSCs and osteoprogenitors, which are important for efficient bone regeneration) from the Bone Marrow Aspirate (BMA) will develop into endothelial and osteoblastic cells, thereby accelerating the healing time of the patient. In various embodiments, the BMA may be centrifuged as part of surgical procedure to increase the concentration of viable cells (Bone Marrow Aspirate Concentrate, BMAC), an effective product in the support of bone regeneration and augmentation of bone grafts.

The second component of the device, autologous plasma-rich platelets (PRP), like the cells in the Bone Marrow Aspirate (BMA), is harvested directly from patient's own venous blood. PRP contains and stimulates the release of endogenous growth factors and proteins such as VEGF (Vascular endothelial growth factor) and TGF- β (Transforming Growth Factor Beta) which stimulate vascularization, angiogenesis, bone repair and regeneration. PRP is a very important faction. As the name suggests it contains a high concentration of platelets - which are cytoplasmic fragments of megakaryocytes - formed in the marrow. Platelets are approximately 2 µm in diameter. The PRP faction contains over 30 bioactive proteins many of which have fundamental role in tissue healing, specifically Platelet Derived Growth Factor (α and β), TGF (α and β), VEGF, Epidermal Growth Factor, Fibroblast Growth Factor, Connective Tissue Growth Factor and Insulin-like Growth Factor I (IGF). All of these growth factors are actively secreted by the platelet and initiate all wound healing processes. In this invention, the growth factors PDGF, BMP, IGF, and TGF-β, will encourage both autologous osteogenic and angiogenic cells to migrate into the scaffold, and induce their proliferation and differentiation. This fraction also contains Fibrin, fibronectin and vitronectin proteins found in the blood which act as adhesion molecules.

Both autologous osteogenic and angiogenic cells and PRP are harvested, applied to the device of the present invention and re-implanted into the patient at point of care during a single surgical procedure. BMAC and PRP will generally be delivered to the target site via a matrix acting as a scaffold which permits cellular (endothelial and osteoblastic) growth and proliferation, with the ultimate goal of accelerating the healing time for the patient.

The composition of the device also allows it to be loaded or compounded during manufacture or at point of care with pharmaceutical or medical devices e.g. bone morphogenetic proteins, antibiotics, antifungals, antivirals, bisphosphonates, growth factors, hormones, proteins and other inorganic minerals such as magnesium etc.

The device may be delivered to the patient either by direct implantation or through a cannulated device to the desired area. An example of this system is the AVN System manufactured by Industrial Technology Systems Ltd. (ITS). This unique system reduces the invasiveness of the surgical procedure and shortens the surgery time in the operating room. This invention in collaboration with the AVN system would be an ideal alternative to total hip replacement by inhibiting the cellular degradation of the femoral head that leads to the destruction of the trabecular structure and its collapse. The methods of implantation are dependent on 1) the anatomical region and 2) the preference of the surgeon.

### BONE PRECURSOR MATERIAL

This invention combines all the elements required to expedite the bone healing process by supplying BMA, PRP's, Growth Factors and a stable mechanical environment (either provided by surgical fixation device or a biomaterial based scaffold).

The third component of the invention includes a biomimetic bone precursor and an osteoconductive scaffold. The biomimetic bone precursor, is bea calcium phosphate such as octacalcium phosphate (OCP), dicalcium phosphate dehydrate (DCPD), or a combination of both. Since the majority of bone consists of endogenous hydroxyapatite, which these chemicals readily convert to, these are considered optimal materials to include within the device. The choice of material is not limited to these; however, it will be confined to materials considered to be biomimetic and a precursor to bone formation. This component will facilitate generation of new bone by readily providing the initial material required for the body to trigger the process and may also contain some additional materials that may enhance the bone repair.

The components of the device each serve a different function in promoting vascularization, angiogenesis, bone repair and regeneration. The biological scaffold (discussed later) provides a structure on which cells may attach prior to implantation to the patient and also during the initial period post-operatively. Over a relatively short period of time within the body, the scaffold will be naturally metabolized, ultimately being completely remodelled through standard biological processes.

Ceramic materials (generally calcium phosphate) are the favoured osteoconductive scaffold when a high mechanical strength environment is required, with Hydroxyapatite (HA) and β-tricaicium phosphate (β-TCP) being well established materials having both biocompatible and osteoconductive properties. These materials are typically remodelled within the body by a cell-mediated response. Depending on the formulation of the scaffold, remodelling may take from months to several years to complete and the high mechanical strength of these materials may inhibit flexibility within the material. Synthetic polymers combine superior mechanical strength with a faster degradation rate within the body, however these may be rejected by the body due to reduced bioactivity.

Tricalcium phosphate (TCP) is one of the most common calcium phosphate biomaterials used to date. TCP acts efficiently as an osteoconductive scaffold. It is more soluble and less crystalline than HA and therefore is more readily resorbed. TCP exists in two forms alpha (α) and beta (β) TCP with α-TCP being more soluble than β-TCP and resorbs faster in *in vivo.*

In most embodiments of the present invention, the biomimetic bone precursor comprises Octacalcium Phosphate (OCP). OCP is a biomimetic bone precursor which has both biocompatible and osteoconductive properties. As Octacalcium Phosphate (OCP) is resorbed, the calcium phosphate material is replaced with newly formed bone. OCP has been confirmed as an osteoconductive material that enhances bone regeneration.

The granule size of the OCP is another important factor to consider in the development of this device. Murakami et at., (Acta Biomater, 2010, Vol 6, Pg. 1542) illustrated in their study that bone regeneration was enhanced with increasing OCP granule size (the largest granules ranging from 500 - 1000 µm). In this study OCP granules were implanted into a critical sized mouse cranial defect. In contrast to this Tanuma etal., (Tissue Engineering, 2012, vol 18, Pg. 546-557) showed that the OCP granules of the smallest size (53 to 300 µm) enhanced both bone regeneration and biodegradation in a critical sized calvaria defect in Wistar rats. In this study, the OCP was mixed with the collagen before it was freeze dried. Therefore depending on the application of the embodiment the size of the OCP may vary. For instance, when the OCP is being dispersed between the two layers of collagen a larger pore size may be necessary but when the OCP is being mixed within the collagen layers, a smaller OCP granule size may be required.

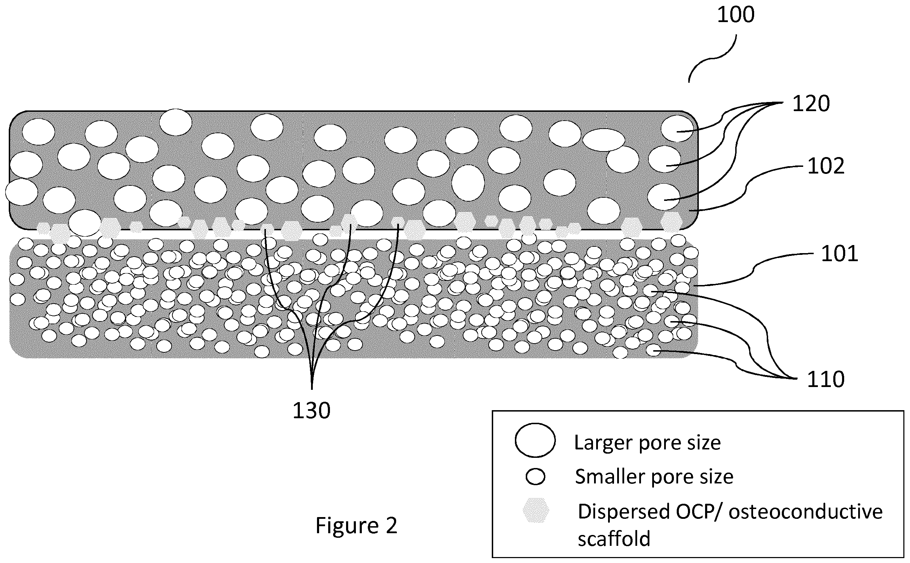

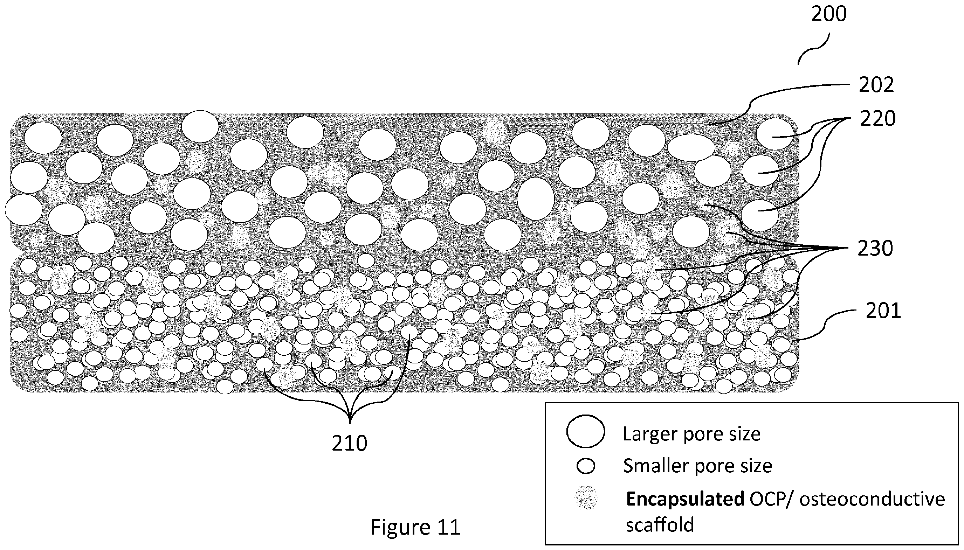

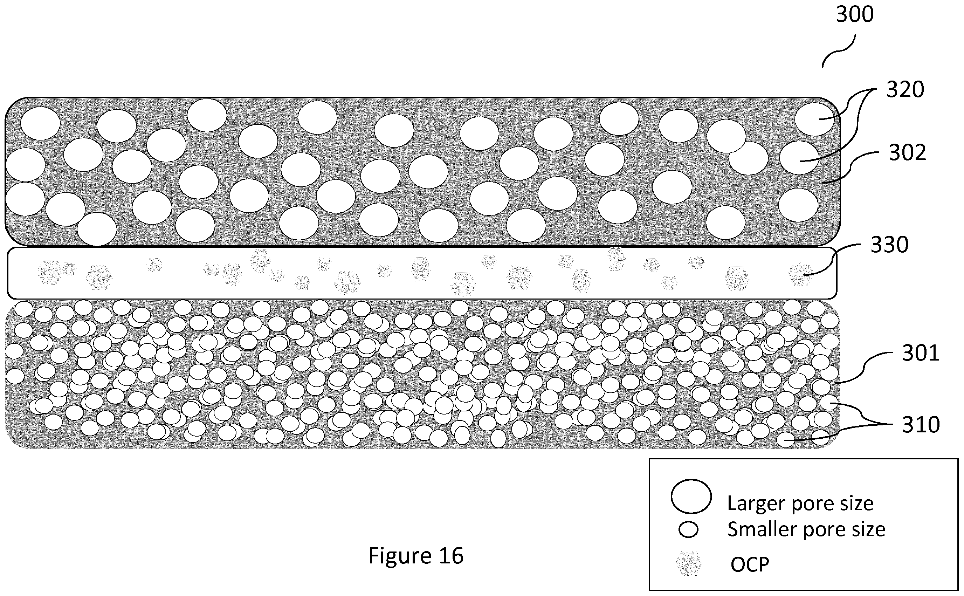

In a preferred embodiment of the device of the present invention, a biomimetic bone precursor calcium salt such as OCP and/or TCP and/or HA and/or DCPD and/or DCPA and/or ACP and/or Calcium Carbonate is encapsulated between each sponge. The composition and dispersion of the calcium phosphate throughout the device may vary depending on the anatomical region to be treated, the injured state of the bone and the target patient population e.g. the calcium phosphate may be evenly distributed throughout each sponge scaffold (Figures 11-14, 17), may be layered (Figures 2-9), or may be sandwiched between (Figure 16).

### OSTEOCONDUCTIVE SCAFFOLD

The Osteoconductive scaffold will be constituted of biomaterials commonly and currently used to make scaffolds. The scaffold component of the device (as illustrated in figure 2) preferably comprises collagen sponges. These sponges are typically produced from bovine or porcine derived collagen but can also be composed of a hydrogel (natural or synthetic) or a mixture of both as required by the therapeutic indication. Osteoconductive scaffolds produced from biological materials (such as bovine or porcine derived collagens) are not recognized for their mechanical strength, however they are easily metabolized by the body and are structurally flexible allowing increased options for the surgeon in their placement. The sponge layers may differ in material and/or specification. This is to allow for optimization of the material to best contain the agents it hosts. The sponge form of the collagen will be highly porous to maximize flexibility and surface area for cell growth. The device is adaptable to permit addition of further materials i.e. pharmaceutical or medical devices e.g. bone morphogenetic proteins, antibiotics, antifungals, antivirals, bisphosphonates, growth factors, hormones, proteins and other inorganic minerals such as magnesium etc.

In most embodiments, collagen will be used, while in other embodiments additional materials for example hydrogel (natural or synthetic) can be used. The utilization of hydrogel in the composition of the scaffold sponges further enhances 3D cellular growth throughout the scaffold.

The characteristics inherent to scaffolds of this nature also facilitate increased cell (osteogenic and angiogenic) growth and proliferation due to a greater surface area.

### PORE SIZE

An important factor to keep in mind, when considering the Osteoconductive scaffold is the pore size. Generally, cells exhibit a preference for adhesion to a porous scaffold with mean pore sizes slightly larger than the cell characteristic size. In terms of the PRP, which have a pore size of 2 µM - the pore size of the scaffold, preferably, a collagen scaffold should be a little larger than 2 µM, but not too big as the first criteria of the collagen is to trap the PRP as the PRP is canted onto the first scaffold component. In terms of bone regeneration, a pore size of 100 - 350 µM is required. MSCs range in size depending on a number of factors including: where they are in their cell cycle (i.e. cells in a G0/G1 phase have a size of 10-13 µM, cells in G2 are between 20 - 25 µM and cells in S-phase fall in between), the density of the cells, the surface they sit on and the age of the cells. In culture, MSC get bigger and fried egg shaped the older they get. Many publications state that scaffolds with a pore size of 325 µM facilitate improved infiltration, earlier expression of mature bone markers, and increased mineralisation. All of which are key factors in the attenuation of bone regeneration and repair.

Porous collagen scaffolds having a uniform pore structure have primarily been manufactured using a freeze drying/ lyophilisation techniques although there are many different techniques which can be used including: solvent casting/particle leeching, gas foaming and electrospinning. A combination of techniques may be required depending on the therapeutic indication.

Larger pore sizes in the scaffold enhances diffusion rates to and from the scaffolds centre, facilitates vascularisation, improves oxygen and nutrient supply as well as efficient waste removal. This environment is more conducive to osteogenesis than constrained scaffolds with smaller pore sizes. In a study by Murphy et al., (Cell adhesion and Migration, 2010, 4:3, 377-381) they noticed cell aggregations along the edges of scaffolds with smaller pore sizes (85 - 120 µM) - which limited the number of cells infiltrating the scaffold. These cell aggregations restricted the diffusion of nutrients and removal of waste from the cells colonising the scaffolds centre.

Keeping in mind the advantages of the larger pore size scaffold however, the mechanical properties of the scaffold may be compromised if the pore sizes are too large. Since the mechanical strength provided by the scaffold decrease with porosity this value should always be balanced with the mechanical needs of the particular tissue that is going to be replaced, which in the case of AVN is a non-load bearing application. This invention is designed to address bone repair and regeneration as well as revascularisation so in this instance mechanical strength is not an important factor.

The device of the present invention comprises a bone precursor material (described previously) supplied between the two sponges or dispersed throughout the scaffold component. The bone precursor material comprises a biomimetic calcium mineral layer. One sponge is optimally designed to carry autologous cells (suspended in bone marrow aspirate) so as to encourage angiogenic and osteogenic growth and proliferation, the other sponge is optimally designed to carry autologous PRP, providing a non-hostile environment for proteins (growth factors and hormones) to reside prior to implantation and to allow interaction with the autologous progenitor cells.

The scaffold sponges are pliable and can be cut and shaped as to allow direct placement at site of the bone or to allow delivery through a cannulated system for example the AVN system ensuring maximum flexibility for treatment of bone defects.

The scaffolds in conjunction with the biological components that they carry and biomimetic mineral they encapsulate, contain all the required components to promote angio and osteogenesis and as such, aid in the treatment and/or prevention of progression of disease states such as avascular necrosis within a bony region.

In summary, the device of the present invention is configured to deliver all four components conceptualised by the "diamond concept" for efficient bone repair whilst additionally adding biological requirements for angiogenesis and vascularisation. A fully biodegradable scaffold encapsulating an inorganic biomimetic bone precursor designed to carry both autologous cells suspended in bone marrow aspirate and PRP that is implantable directly or deliverable through a cannulated system to the site of an injured bone. The materials are optimized to best facilitate cellular growth within the scaffold and to best house the proteins contained within the PRP. By introducing the device of the present invention to a properly treated site e.g. debridement to remove all necrotic tissue, the device has the advantage of delivering all the components capable of stimulating vascularization, angiogenesis, bone repair and regeneration and preventing progression of diseases states such as avascular necrosis within a bone, therefore considerably reducing the potential for more invasive surgical interventions such as a total hip replacement.

### Brief Description Of The Drawings

The device of the present invention will now be described more particularly, with reference to the accompanying drawings and to the following Examples, in which are, respectively, shown and described, by way of example only, a number of alternative embodiments of the device and method of the present invention.

In the drawings:
Figure 1 is a schematic diagram showing the requirements for bone regeneration and revascularisation represented by the "Diamond Concept";
Figure 2 is a schematic longitudinal cross sectional view of a first embodiment of the device of the present invention and more specifically, represents a first stage in the manufacture of a first embodiment of the device of the present invention comprising:
   A first scaffold component comprising a porous biological material comprising, in this embodiment, collagen, configured to carry and maintain growth factors, for instance, growth factors present in BMA and/or viable platelet rich plasma (PRP);
   A second scaffold component comprising a porous biological material comprising, in this embodiment, collagen, configured to carry and maintain viable osteogenic and angiogenic cells provided by Bone Marrow Aspirate (BMA); and
   A third component comprising a biomimetic bone generating phase, optionally comprising a calcium phase; which also functions as an osteoconductive scaffold for providing a stable mechanical environment. In this embodiment, the biomimetic calcium phase preferably comprises a calcium phosphate compound optionally, in the form of a salt. In this embodiment, the calcium phosphate compound comprises Octacalcium Phosphate (OCP);

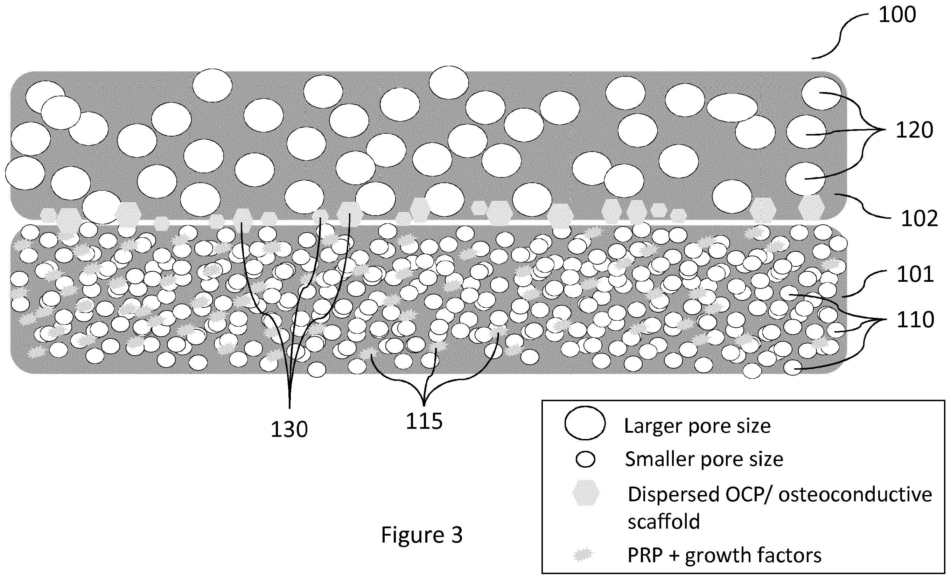

Figure 3 is a schematic longitudinal cross sectional view of the first embodiment of the device of the present invention shown in Figure 2, and more specifically, represents a stage in the in-situ use of the first embodiment of the device of the present invention as will be described hereinbelow;

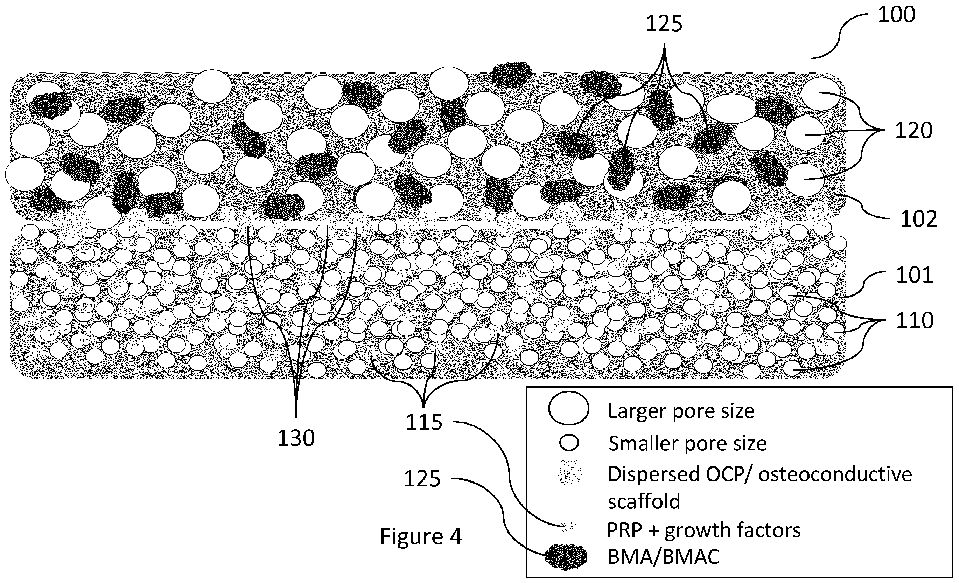

Figure 4 is a schematic longitudinal cross sectional view of the first embodiment of the device of the present invention shown in Figure 2, and more specifically, represents a stage in the in-situ use of the first embodiment of the device of the present invention;

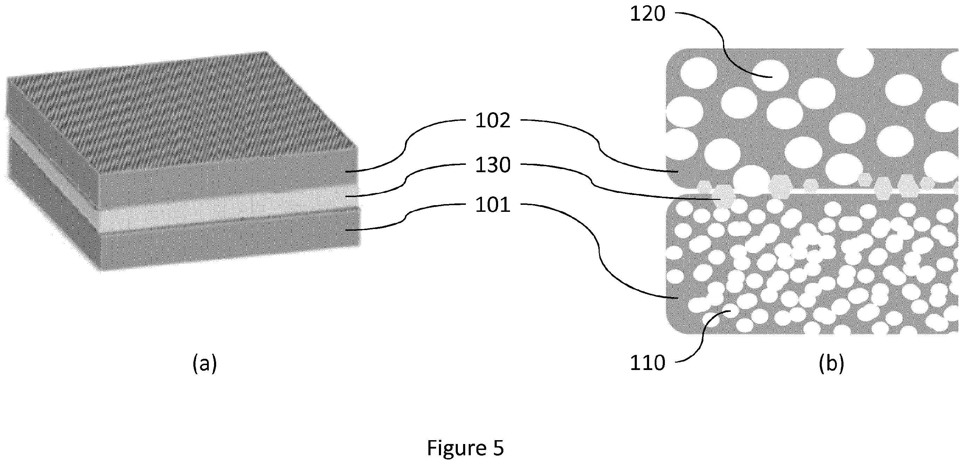

Figure 5 is a schematic diagram showing the Figures of the earlier patent application and the related Figures of the present application;

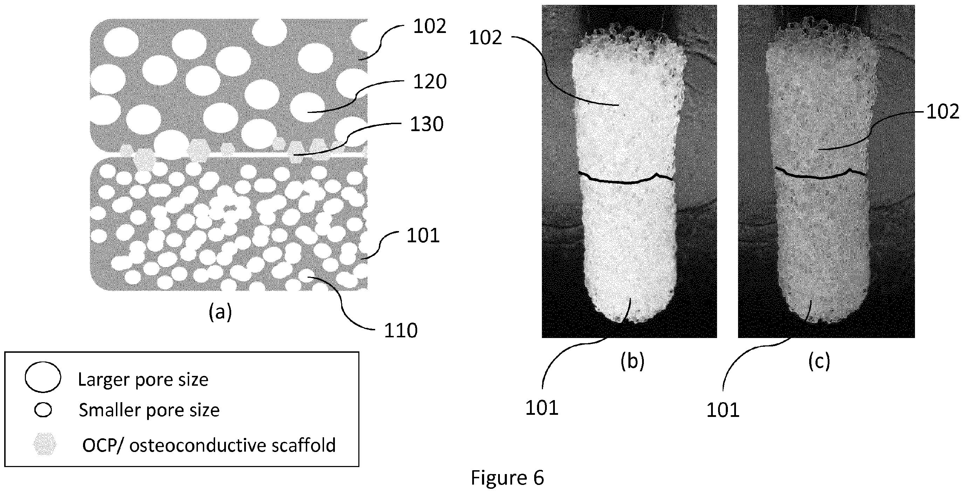

Figure 6(a) is the same as shown in Figure 2 and Figures 6(b) and (c) are photos showing alternative views of the device of the first embodiment and showing in particular, the porous nature of each of the biological material scaffolds;

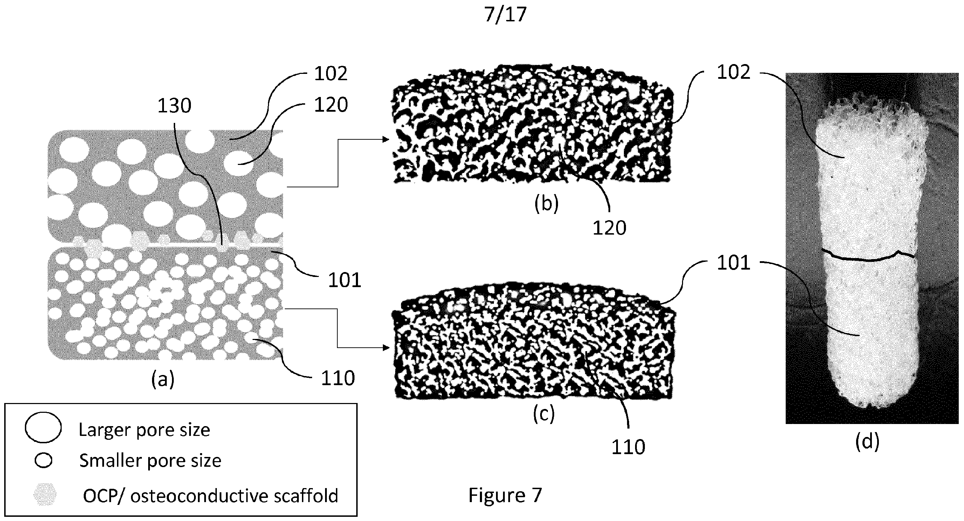

Figure 7 is a similar view to that shown in Figures 6 (a), (b) and (c);

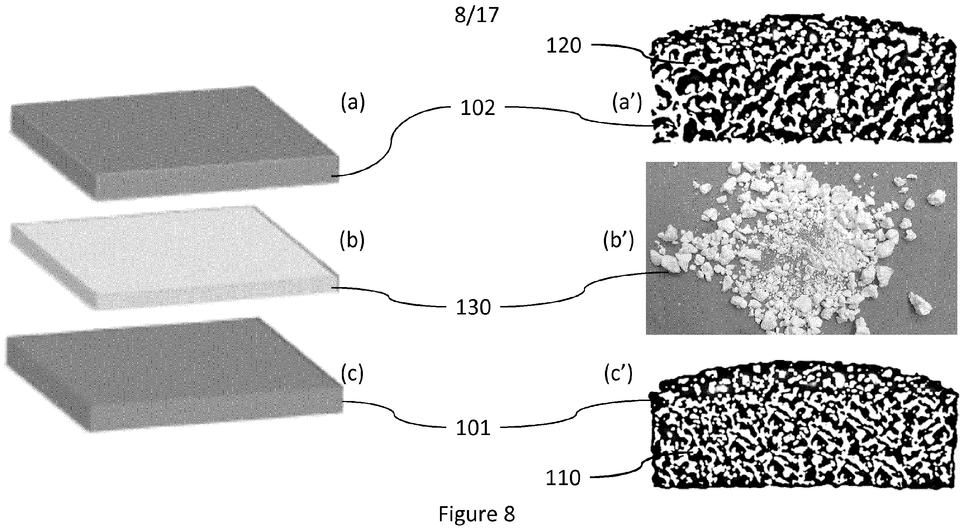

Figure 8 shows in cross section, the porous nature of the respective scaffold sponges, showing in particular, the relatively larger pore size of the second scaffold sponge and the relatively smaller pore size of the first scaffold sponge; the granular nature of the OCP is also shown in Figure 8;

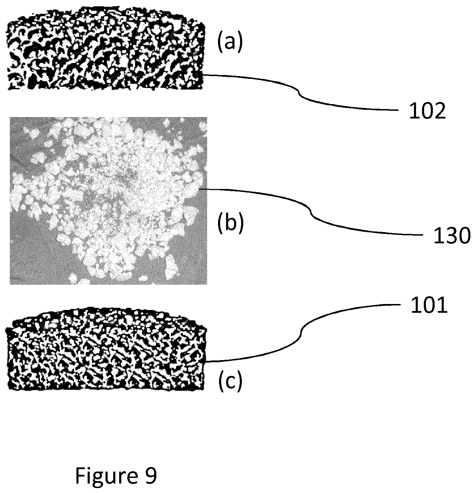

Figure 9 is a further view similar to that shown in Figure 8;

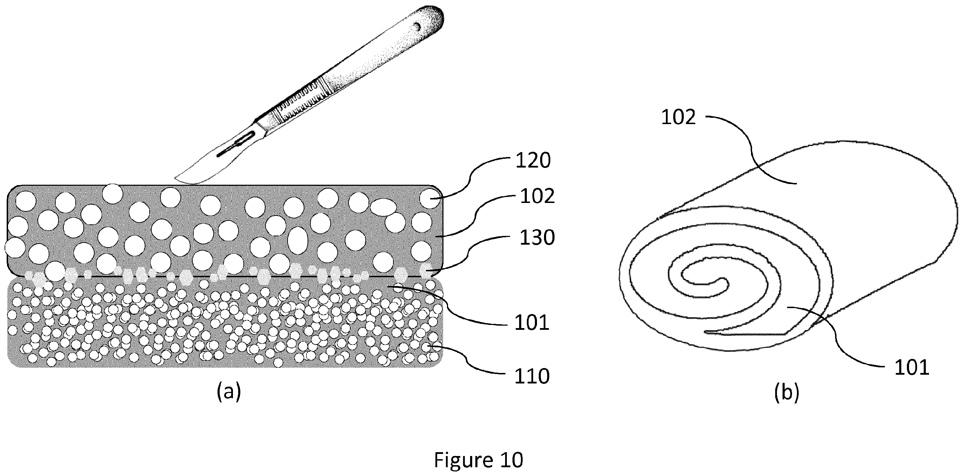

Figure 10 is a schematic diagram showing the manner of use of the device shown in Figures 1 to 10;
Figure 11 is a cross sectional view of an alternative embodiment of the device of the present invention in which the OCP is encapsulated in the scaffold sponge and are interspersed throughout the collagen layer(s); not dispersed in between the first scaffold sponge and the second scaffold sponge as was the case in the first embodiment shown in Figures 1 to 10; as the collagen layers are being cast, the OCP granules will be mixed into the collagen before it solidifies so that the granules are interspersed throughout the collagen layer.

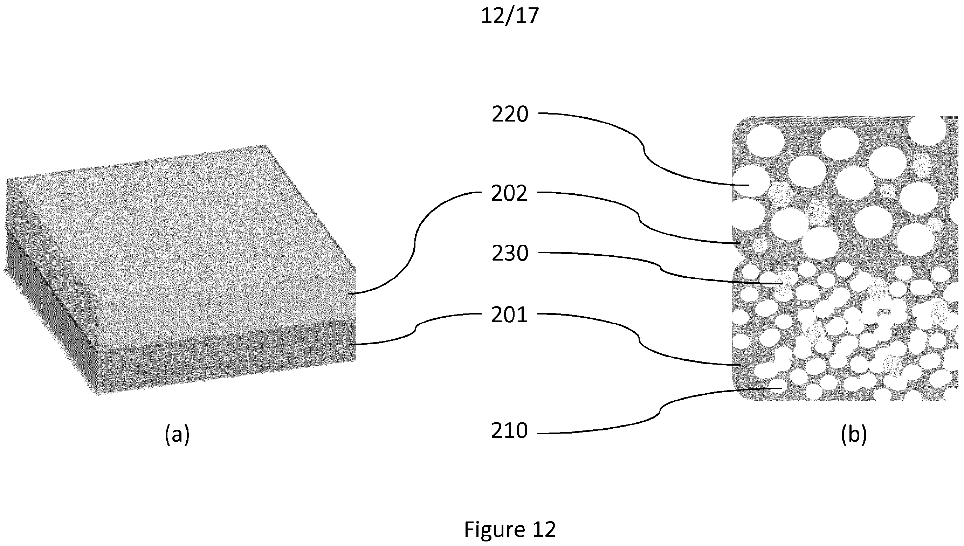

Figure 12 is a schematic diagram showing the Figures of the earlier patent application and the Figures of the present application in relation to the second embodiment;

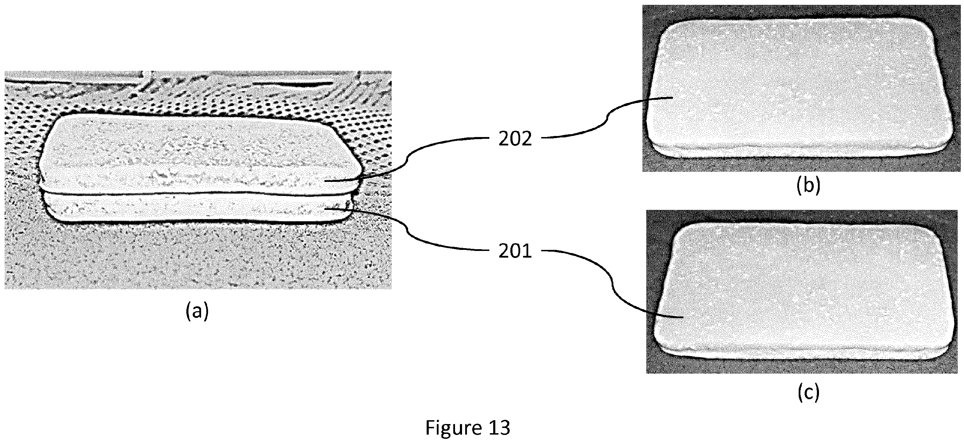

Figure 13(a), Figure 13(b) and 13(c) are pictorial representations showing alternative views of the device of the alternative embodiment of Figures 11 and 12;

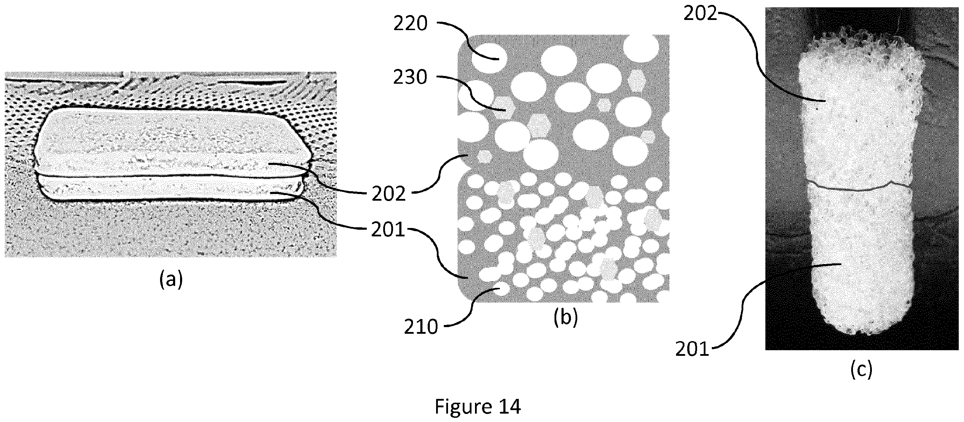

Figure 14(a), Figure 14(b) and Figure 14(c) are pictorial representations of the device of the alternative embodiment of Figures 11 to 13 and Figure 14(c) is a photo of the device as shown in Figures 14(a) and 14(b);

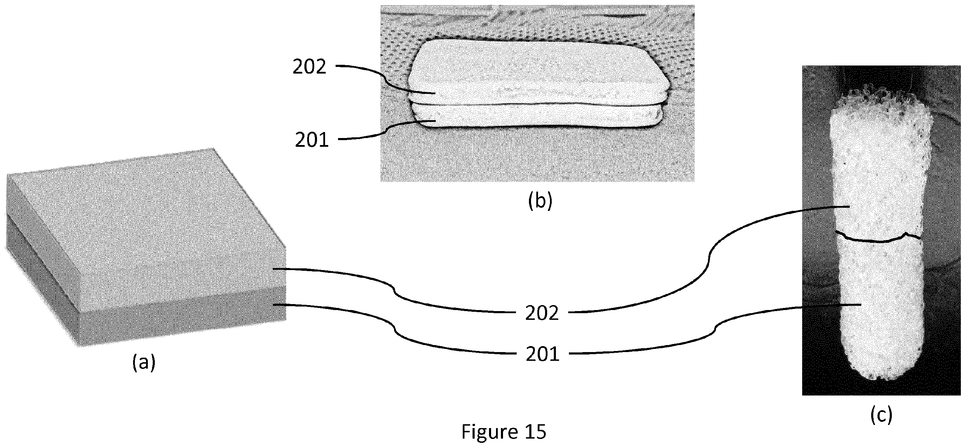

Figure 15(a), Figure 15(b) and Figure 15(c) show the same device and views as shown in Figures 14(a), Figure 14(b) and Figure 14(c);
Figure 16 is a schematic diagram of an alternative embodiment where the OCP is encapsulated within a third collagen layer which is "sandwiched" between the first and second collagen layers; with a porosity to specifically trap the PRP fraction and associated growth factors. The second collagen layer is designed specifically for the BMA/BMAC cells; and

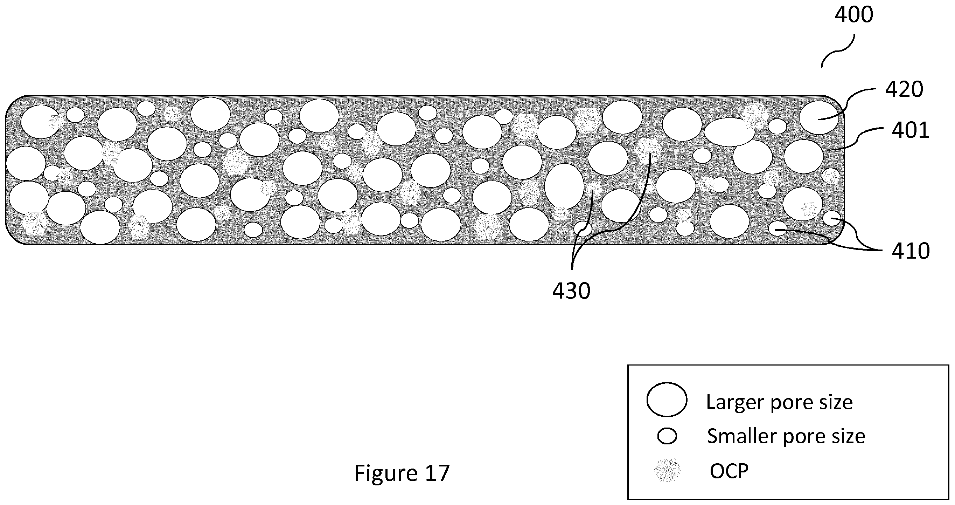

Figure 17 is a schematic diagram of a further alternative embodiment comprising a single collagen sponge comprising both the relative large pores (100 - 350 µM) and the relative small pores (2 - 10 µM) and also comprising the OCP encapsulated within one single collagen layer.

### Detailed Description Of The Drawings

Referring initially to Figures 2 to 10, there is shown a first embodiment of the device of the present invention. The device in this embodiment is indicated generally by the reference numeral 100 and comprising a first porous scaffold component and a second porous scaffold component. The first scaffold component is indicated generally by reference numeral 101 and comprises pores 110 having a relatively small pore size (2 - 10 µM). The second scaffold component 102 comprises pores 120 having a relatively larger pore size (100 - 350 µM).

This sizing of the pores 110, 120 in the respective first scaffold component 101 and the second scaffold 102 has the advantage that the relatively larger pore size of the second scaffold sponge 102 is adapted for holding and delivering the osteogenic and/or angiogenic cells to the bone defect site; and the relatively smaller pore size of the first scaffold sponge 101 is adapted for holding and delivering the PRP and growth factors to the bone defect site.

As also shown in Figures 2 to 10, a biomimetic bone generation agent, preferably, OCP is provided in between the first scaffold component 101 and the second scaffold component 102. The OCP is preferably in the form of granules 130 as shown in Figures 2 and corresponding Figures and also shown in Figure 8b'. The granules 130 of OCP are dispersed on the surface of the first scaffold component 101 which in this embodiment is a porous sponge 101 and the second scaffold component 102 which is also a porous sponge 102.

As shown in Figure 3, growth factors provided by Plasma-rich platelets (PRP) are included in the first sponge and as shown in Figure 4, osteogenic and/or angiogenic cells are incorporated in the second scaffold sponge 102.

Referring now to Figure 11, there is shown, a second embodiment of the device of the present invention in which the OCP indicated by reference numeral 230 is encapsulated in the scaffold sponge and are interspersed throughout the collagen layer(s); thus, in this second embodiment, the OCP is not dispersed in between the first scaffold sponge and the second scaffold sponge as was the case in the first embodiment shown in Figures 1 to 10; as the collagen layers are being cast, the OCP granules 230 will be mixed into the collagen before it solidifies so that the OCP granules 230 are interspersed throughout the collagen layer.

The device in the second embodiment is indicated generally by the reference numeral 200 and comprising a first porous scaffold component and a second porous scaffold component. The first scaffold component is indicated generally by reference numeral 201 and comprises pores 210 having a relatively small pore size of 2-10 µM. The second scaffold component 202 comprises pores 220 having a relatively larger pore size of 100 - 350 µM.

This sizing of the pores 210, 220 in the respective first scaffold component 101 and the second scaffold 202 has the advantage that the relatively larger pore size of the second scaffold sponge 202 is adapted for holding and delivering the osteogenic and/or angiogenic cells to the bone defect site; and the relatively smaller pore size of the first scaffold sponge 201 is adapted for holding and delivering the growth factors to the bone defect site.

Referring now to Figure 16, an alternative embodiment of the device will be described. The device in this embodiment is indicated generally by the reference numeral 300 and comprising a first porous scaffold component and a second porous scaffold component. The first scaffold component has a relatively small pore size of 2 - 10 µM and the second scaffold component has a relatively larger pore size of 100 - 350 µM and where the OCP, in the form of granules 330, is encapsulated within a third scaffold layer which is "sandwiched" between the first and second porous scaffold layers; the first porous scaffold component comprising a porosity to specifically trap the PRP fraction and associated growth factors. The second collagen layer 302 is designed specifically for the BMA/BMAC cells.

Thus the device 300 comprising a first porous scaffold component 301 and a second porous scaffold component 302. The first scaffold component 301 comprises pores 310 having a relatively small pore size of 2 - 10 µM. The second scaffold component 302 comprises pores 320 having a relatively larger pore size of 100 - 350 µM.

This sizing of the pores 310, 320 in the respective first scaffold component 301 and the second scaffold 302 has the advantage that the relatively larger pore size of the second scaffold sponge 302 is adapted for holding and delivering the osteogenic and/or angiogenic cells to the bone defect site; and the relatively smaller pore size of the first scaffold sponge 301 is adapted for holding and delivering the growth factors to the bone defect site.

Referring now to Figure 17, a further alternative embodiment of the device will be described. The device in this embodiment is indicated generally by the reference numeral 400 and comprising a single scaffold component in the form of a collagen sponge 401 comprising both the relatively large pores 420 (pore size in the range of 100 - 350 µM) and the relatively small pores 410 (pore size in the range of 2 - 10 µM) and also comprising the OCP 430 encapsulated within the single collagen layer.

### EXAMPLES:

### EXAMPLE 1

OCP is prepared following a modification of the publication LeGeros (Calcif Tissue Int. 1985 Mar; 37(2):194-7) in which calcium and phosphate solutions are first prepared and then mixed. Once the precipitate is fully formed, the OCP is filtered and heated to 50°C to evaporate any remaining liquids. Once dried, the OCP precipitate is passed through standard testing sieves. The granules are separated into 3 groups, including granules with diameters ranging from 53 to 300 µm, 300 to 500 µm and 500 to 1000 µm. These are sterilised by heating at 120°C for 2 hours. Previous research has shown that this treatment of heating to 120°C for 2 hours does not affect the physical properties such as the crystalline structure or specific surface are of the OCP granules. It has also been reported that temperatures exceeding 100°C, over time, induce a gradual collapse of the OCP structure due to dehydration.

The collagen can be cast using either freeze dry or electrospinning techniques. In this Example, Collagen scaffolds will be produced by lyophilisation of a collagen suspension. The suspension will be produced by blending micro fibrillar bovine tendon collagen in 0.05 M acetic acid. The resulting suspension will contain 0.5% w/v of collagen. The suspension will be maintained at 4°C during blending to prevent denaturation of the collagen. Following blending the suspension will be degassed in a vacuum desiccator for 60 min to remove trapped air bubbles. The Collagen slurry will be lyophilized following a protocol developed by O'Brien et al., to produce Collagen scaffolds with a homogeneous pore structure. Briefly, 67.25 mL of the Collagen suspension will be pipetted into a stainless-steel tray (5x5 in., grade 304 SS). The tray will be placed onto the freeze-dryer shelf and the freezing cycle will be started. To make the collagen with the larger pore size i.e. 325 µM - the samples will be cooled to a temperature of -20C at a constant cooling rate of 0.9°C=min and will be held at this temperature for 20 min to allow the suspension to freeze. The temperature will then be increased to -10°C to start annealing and will be held there for a specific annealing time which in this case is 0.25 hours. After freeze-drying the scaffolds will be dehydrothermally crosslinked at 105°C for 24 hours in a vacuum oven at 50 mTorr (1 Torr corresponds to 133.3 Pa).

In one embodiment of the present invention, the collagen is cast with conditions to produce a small pore size (e.g. varying the concentration of acetic acid), freeze dried, and OCP with a granular size of 500 to 1000 µM will be added - not in a layer but rather as granules dispersed on top of the set collagen layer - and the second collagen layer will be cast on top and freeze dried at different conditions to yield a larger pore size (see above).

In another embodiment of the invention - the first collagen layer (smaller pore scaffold) and the second collagen layer (larger pore scaffold) will be cast separately. Again, the OCP granules will be dispersed on top of one of the layers, then using a biological adhesive and/or a synthetic glue, the first and second layers will be glued together.

In yet another embodiment of this invention - as the collagen layers are being cast, the OCP granules (53 - 300 µM) will be mixed into the collagen before it solidifies so that the granules are interspersed throughout the collagen layers.

Once the surgeon has the device in the operating theatre, the scaffold can be cut to the required size depending on the therapeutic application. For this example, the collagen scaffolds can be cut into strips of 5 x 3 cm.

### Plasma-rich platelets (PRP)

The PRP is obtained from a sample of patients' blood drawn during surgery. PRP is produced by centrifugation of whole blood using the Smartprep2^{™} centrifuge (Harvest Technologies, Munich, Germany). In general, the blood is centrifuged twice - the first spin, separates out the Red blood cells (RBC) while the second centrifugation step concentrates the Platelets in the smallest final plasma volume. An initial starting volume of 30 cc venous blood draw will yield 3 - 5 cc of PRP.

This obtained volume of PRP will then be canted over the top of the device allowing a few minutes for the PRP to sediment down through the collagen scaffold and for the scaffold to absorb the PRP and all essential elements e.g. growth factors.

### BONE MARROW ASPIRATE (BMA)

In some embodiments of this invention, BMA will be used and in other embodiments of the invention, BMAC will be used. Both techniques are described here.

A total of 60 ml bone marrow aspirate (BMA) is obtained by vacuum aspiration from the posterior iliac crest. Bone marrow aspiration concentrate (BMAC) is produced via density gradient centrifugation using the Smartprep2^{™} centrifuge (Harvest Technologies, Munich, Germany) in accordance with the manufacturer's directions including 2,500 rpm for 3 min and followed by 2,300 rpm for 9 min to yield a total volume of 8 ml BMAC.

This volume of BMAC is carefully canted over the top of the already soaked device allowing a few minutes for the BMAC to sediment down through the collagen scaffold and also allowing the scaffold to absorb the BMAC.

Once the scaffold has absorbed both cell types the surgeon can roll the strip into a roll as shown schematically, in Figure 10 and using the AVN system the device of the present invention can be implanted into the femoral head. Several strips/rolls can be used to fill the bone void.

### EXAMPLE 2

Again, OCP will be prepared following a modification of the publication LeGeros (Calcif Tissue Int. 1985 Mar; 37(2):194-7) in which calcium and phosphate solutions are first prepared and then mixed. Once the precipitate is formed, the OCP is filtered and heated to 50°C to evaporate any remaining liquids. Once dried, the OCP precipitate is passed through standard testing sieves. Granules are separated into 3 groups, granules with diameters ranging from 53 to 300 µm, 300 to 500 µm and 500 to 1000 µm. These will be sterilised by heating at 120°C for 2 hours. Previous research has shown that this treatment of heating to 120°C for 2 hours does not affect the physical properties such as the crystalline structure or specific surface are of the OCP granules. It has also been reported that temperatures exceeding 100°C, overtime induce a gradual collapse of the OCP structure due to dehydration.

In this embodiment of the Invention, Spinplant in Germany which can produce collagen Nano fibres will design two collagen weaves specifically designed to trap either BMA/BMAC or PRPs depending on their pore sizes. As with Example 1, the smaller PRP cells will be initially canted onto the device in surgery followed by the larger BMAC, allowing time for them to sediment through the collagen scaffold. The scaffolds designed by Spinplant will be optimized to best facilitate cellular growth within the scaffold and to best house the proteins contained within the PRP.

Once the surgeon has the device in the operating theatre, the scaffold can be cut to the required size depending on the therapeutic application. For this example, the collagen scaffolds can be cut into strips of 5 x 3 cm.

### Plasma-rich Platelets (PRP)

The PRP is obtained from a sample of patients' blood drawn during surgery. PRP is produced by centrifugation of whole blood using the Smartprep2^{™} centrifuge (Harvest Technologies, Munich, Germany). In general, the blood is centrifuged twice - the first spin, separates out the Red blood cells (RBC) while the second centrifugation step concentrates the Platelets in the smallest final plasma volume. An initial starting volume of 30 cc venous blood draw will yield 3 - 5 cc of PRP.

This obtained volume of PRP will then be canted over the top of the device allowing a few minutes for the PRP to sediment down through the collagen scaffold and for the scaffold to absorb the PRP and all essential elements e.g. growth factors.

### BONE MARROW ASPIRATE

In some embodiments of this invention, BMA is used and in other embodiments of the invention, BMAC are used. Both techniques are described here.

A total of 60 ml bone marrow aspirate (BMA) is obtained by vacuum aspiration from the posterior iliac crest. Bone marrow aspiration concentrate (BMAC) is produced via density gradient centrifugation using the Smartprep2^{™} centrifuge (Harvest Technologies, Munich, Germany) in accordance with the manufacturer's directions including 2,500 rpm for 3 min and followed by 2,300 rpm for 9 min to yield a total volume of 8 ml BMAC.

This volume of BMAC is carefully canted over the top of the already soaked device allowing a few minutes for the BMAC to sediment down through the collagen scaffold and also allowing the scaffold to absorb the BMAC.

Once the scaffold has absorbed both cell types, the surgeon can roll the strip into a roll and, using the AVN system, the device of the present invention can be implanted into the femoral head. Several strips/rolls can be used to fill the bone void.

In some embodiments, the BMA/BMAC may be collected preoperatively, grown/modified in the lab and used when needed - during surgery. This changes the categorisation of the invention from a medical device to a medicine.

In some embodiments, the damaged tissue/bone may require surgical intervention/stabilisation in addition to the materials added to address the bone defect and aid in the bone regeneration. For example, in some embodiments once the device has been implanted, secure pins, screws and other orthopaedic devices can be used to fix the bone fracture in place.

In some embodiments, different sized β-TCP/OCP granules may be used to trap air bubbles between the two sheets of collagen to be utilised by the cells as needed.

In some embodiments, the collagen sheets will be flushed with oxygen so that oxygen will be trapped within the fibres and can be utilised by the cells when needed.

In some embodiments as the collagen layer is being made the step to remove trapped air bubbles specifically degassing in a vacuum desiccator for 60 min will be eliminated. These trapped air bubbles can be utilised by the cells if needed.

The words comprises/comprising when used in this specification are to specify the presence of stated features, integers, steps or components but does not preclude the presence or addition of one or more other features, integers, steps, components or groups thereof.

The examples and other embodiments contained here are only exemplary in nature. They and are not intended to be limiting in describing the full scope of compositions of this invention as defined in the appended claims.

## Claims

1. An implantable device configured to deliver, to an injured bone site, components for revascularisation and bone repair, and designed to treat Avascular Necrosis (AVN) and aid in musculo-skeletal tissue healing, the device comprising: a first osteoconductive scaffold component comprising growth factors present in platelet-rich plasma and bone marrow aspirate, for inducing cellular events that initiate healing; and comprising a porous biomaterial of which the pores of the biomaterial have a relatively small pore size, of 2 - 10 µM, sized to hold and deliver to the injured bone site, the growth factors present in the platelet-rich plasma and bone marrow aspirate; and comprising a second osteoconductive scaffold component comprising viable autologous osteogenic and/or angiogenic cells present in bone marrow aspirate; and comprising a porous biomaterial of which the pores of the biomaterial have a relatively larger pore size of 100 - 350 µM sized to hold and deliver to the injured bone site, the viable autologous osteogenic and/or angiogenic cells present in the bone marrow aspirate, and wherein the device also comprises a third scaffold component comprising a biomimetic bone generating agent, wherein the biomimetic bone generating agent comprises an inorganic bone precursor, and wherein the inorganic bone precursor comprises a calcium phosphate based compound that promotes bone cell proliferation and vascularity, and whereby the scaffold components provide a stable mechanical environment for promoting bone cell proliferation and vascularity.

2. A device as claimed in claim 1 wherein the porous biomaterial comprises a sponge; preferably, comprising a natural or synthetic hydrogel matrix for supporting 3D cellular growth throughout the scaffold component; and most preferably, comprises collagen, optionally, in the form of a layer of collagen.

3. A device as claimed in 1 wherein the calcium based compound comprises a biomimetic octacalcium phosphate compound; preferably, provided in granular form and having a particle size that is optimized for cell proliferation and differentiation to osteoblasts, wherein the granules include granules having diameter sizes selected from one of the following diameter ranges: from 53 - 300 µm, 300 - 500 µm and 500 to 1000 µm.

4. A device in claim 1 wherein the biomimetic bone generating agent (bone precursor) comprises a calcium phosphate based salt.

5. A device as claimed in any one of claims 1 to 4 wherein the biomimetic bone generation phase comprises a calcium phase comprising any one or more selected from the following group of calcium compounds: octacalcium phosphate (OCP) and/or tri-calcium phosphate (TCP) and/or hydroxyapatite (HA) and/or dicalcium phosphate dihydrate (DCPD) and/or dicalcium phosphate anhydrous (DCPA) and/or amorphous calcium phosphate (ACP) and/or calcium carbonate.

6. A device as claimed in claim 1 wherein the biomimetic bone generating agent is encapsulated within at least one scaffold component.

7. A device as claimed in any one of claims 1 to 5 wherein the biomimetic bone generating agent is provided between the first scaffold component and the second scaffold component and preferably wherein the biomimetic bone generating agent is provided as a layer between the first scaffold component and the second scaffold component.

8. A device as claimed in any one of the preceding claims wherein the or each osteoconductive scaffold comprises a biological material, preferably a porous biomaterial preferably wherein the biomaterial is selected from one or more of the following: allograft or xenograft trabecular bone, demineralised bone matrix (DBM), collagen, hydroxyapatite (HA), polylactic or polyglycolic acid, bioactive glasses, calcium-based ceramics, and hydrogel or wherein the biomaterial is selected from one or more of the following: collagen and hydrogel.

9. A device as claimed in claim 1 - 2 wherein the scaffold component(s) comprise collagen sheets and wherein the scaffold component(s) are flushed with oxygen so that, air bubbles are trapped between the first and second scaffold components, by different sized B-TCP/OCT granules, or the air bubbles are trapped within the fibres of the collagen, to be utilised by the cells as needed.

10. A device as claimed in any of claims 1 to 5 wherein the biomimetic bone pre-cursor comprises octacalcium phosphate (OCP) in granular form, in combination with dicalcium phosphate dihydrate, with a particle size optimized for cell proliferation.

## Patentansprüche

1. Implantierbare Vorrichtung, die so gestaltet ist, dass sie an eine Knochenverletzungsstelle Komponenten zur Revaskularisierung und Knocheninstandsetzung zuführt, und wobei die Vorrichtung zur Behandlung Avaskulärer Nekrose (AVN) und zur Unterstützung der Heilung von Gewebe des Bewegungsapparates gestaltet ist, wobei die Vorrichtung folgendes umfasst: eine erste osteokonduktive Gerüstkomponente, die in plättchenreichem Plasma vorhandene Wachstumsfaktoren und Knochenmarkaspirat umfasst, zum Induzieren von zellulären Vorgängen, welche die Heilung initiieren; und umfassend ein poröses Biomaterial, wobei die Poren des Biomaterials eine verhältnismäßig kleine Porengröße von 2 bis 10 µM aufweisen, die so bemessen ist, dass sie die in in plättchenreichem Plasma vorhandenen Wachstumsfaktoren und Knochenmarkaspirat halten und an die Knochenverletzungsstelle zuführen; und umfassend eine zweite osteokonduktive Gerüstkomponente, die in Knochenmarkaspirat vorhandene lebensfähige autologe osteogene und/oder angiogene Zellen umfasst, und umfassend ein poröses Biomaterial, wobei die Poren des Biomaterials eine im Verhältnis größerer Porengröße von 100 bis 350 µM aufweisen, die so bemessen ist, dass sie die in dem Knochenmark vorhandenen lebensfähigen autologen osteogenen und/oder angiogenen Zellen halten und an die Knochenverletzungsstelle zuführen, und wobei die Vorrichtung ferner eine dritte Gerüstkomponente umfasst, die eine biomimetische knochenbildende Substanz umfasst, wobei die biomimetische knochenbildende Substanz einen anorganischen Knochenpräkursor umfasst, und wobei der anorganische Knochenpräkursor eine Verbindung auf Kalziumphosphatbasis umfasst, welche die Knochenzellenproliferation und Vaskularität fördert, und wobei die Gerüstkomponenten eine stabile mechanische Umgebung zur Förderung der Knochenzellenproliferation und Vaskularität bereitstellen.

2. Vorrichtung nach Anspruch 1, wobei das poröse Biomaterial einen Schwamm umfasst; vorzugsweise umfassend eine natürliche oder synthetische Hydrogelmatrix zur Unterstützung von dreidimensionalem zellulären Wachstum durch die Gerüstkomponente; und am meisten bevorzugt umfassend Kollagen, optional in Formal einer Kollagenschicht.

3. Vorrichtung nach Anspruch 1, wobei die Verbindung auf Kalziumbasis eine biomimetische Octakalziumphosphatverbindung umfasst; vorzugsweise bereitgestellt in granulärer Form und mit einer optimierten Teilchengröße für die Zellproliferation und Differenzierung zu Osteoblasten, wobei die Körner Körper mit Durchmessergrößen aufweisen, die aus einem der folgenden Durchmesserbereiche ausgewählt sind: von 53 bis 300 µM, von 300 bis 500 µM und von 500 bis 1000 µM.

4. Vorrichtung nach Anspruch 1, wobei die biomimetische knochenbildende Substanz (Knochenpräkursor) ein Salz auf Kalziumphosphatbasis umfasst.

5. Vorrichtung nach einem der Ansprüche 1 bis 4, wobei die Phase der biomimetischen Knochenbildung eine Kalziumphase umfasst, die eines oder mehrere der aus der folgenden Gruppe von Kalziumverbindungen ausgewählten umfasst: Octakalziumphosphat (OCP) und/oder Tri-Kalziumphosphat (TCP) und/oder Hydroxyapatit (HA) und/oder Di-Kalziumphosphatdihydrat (DCPD) und/oder Di-Kalziumphosphatanhydrid (DCPA) und/oder amorphes Kalziumphosphat (ACP) und/oder Kalziumcarbonat.

6. Vorrichtung nach Anspruch 1, wobei die biomimetische knochenbildende Substanz in mindestens einer Gerüstkomponente gekapselt ist.

7. Vorrichtung nach einem der Ansprüche 1 bis 5, wobei die biomimetische knochenbildende Substanz zwischen der ersten Gerüstkomponente und der zweiten Gerüstkomponente bereitgestellt ist, und wobei die biomimetische knochenbildende Substanz vorzugsweise als eine Schicht zwischen der ersten Gerüstkomponente und der zweiten Gerüstkomponente bereitgestellt ist.

8. Vorrichtung nach einem der vorstehenden Ansprüche, wobei das eine oder jedes osteokonduktive Gerüst eine biologische Substanz umfasst, vorzugsweise ein poröses Biomaterial, wobei das Biomaterial vorzugsweise aus einem oder mehreren der folgenden ausgewählt ist: Allograft oder Xenograft Spongiosa, demineralisierter Knochenmatrix (DBM), Kollagen, Hydroxyapatit (PA), Polymilch- oder Polyglykolsäure, bioaktiven Gläsern, Keramikwerkstoffen auf Kalziumbasis und Hydrogel, oder wobei das Biomaterial aus einem oder mehreren der folgenden ausgewählt ist: Kollagen und Hydrogel.

9. Vorrichtung nach einem der Ansprüche 1 bis 2, wobei die Gerüstkomponente(n) Kollagenlagen umfasst/umfassen, und wobei die Gerüstkomponente(n) mit Sauerstoff gespült wird/werden, so dass Luftblasen zwischen den ersten und zweiten Gerüstkomponenten eingeschlossen werden, durch B-TCP/OCT-Körner unterschiedlicher Größen, oder wob ei die Luftblasen in den Fasern des Kollagens eingeschlossen werden, zur Verwendung durch die Zellen nach Bedarf.

10. Vorrichtung nach einem der Ansprüche 1 bis 5, wobei der biomimetische Knochenpräkursor Octakalziumphosphat (OCP) in granulärer Form in Kombination mit Di-Kalziumphosphatdihydrat umfasst, mit einer für die Zellproliferation optimierten Teilchengröße.

## Revendications

1. Dispositif implantable conçu pour administrer, à un site osseux lésé, des composants pour une revascularisation et une réparation osseuse, et conçu pour traiter la nécrose avasculaire (AVN) et aider à la guérison des tissus musculo-squelettiques, le dispositif comprenant : un premier composant de matrice ostéoconductrice comprenant des facteurs de croissance présents dans le plasma riche en plaquettes et l'aspirat de moelle osseuse, pour induire des événements cellulaires qui amorcent la guérison ; et comprenant un biomatériau poreux dont les pores ont une taille de pore relativement petite, de 2 à 10 µM, dimensionnés pour contenir et administrer au site osseux lésé les facteurs de croissance présents dans le plasma riche en plaquettes et l'aspirat de moelle osseuse ; et comprenant un deuxième composant de matrice ostéoconductrice comprenant des cellules ostéogéniques et/ou angiogéniques autologues viables présentes dans l'aspirat de moelle osseuse ; et comprenant un biomatériau poreux dont les pores ont une taille de pore relativement plus grande comprise entre 100 et 350 µM, dimensionné pour contenir et administrer au site osseux lésé les cellules ostéogéniques et/ou angiogéniques autologues viables présentes dans l'aspirat de moelle osseuse, et le dispositif comprenant également un troisième composant de matrice comprenant un agent générateur d'os biomimétique, l'agent générateur d'os biomimétique comprenant un précurseur d'os inorganique, et le précurseur d'os inorganique comprenant un composé à base de phosphate de calcium qui favorise la prolifération des cellules osseuses et la vascularisation, et moyennant quoi les composants de matrice fournissent un environnement mécanique stable pour favoriser la prolifération des cellules osseuses et la vascularisation.

2. Dispositif selon la revendication 1, le biomatériau poreux comprenant une éponge ; de préférence, comprenant une matrice d'hydrogel naturel ou synthétique pour soutenir la croissance cellulaire 3D à travers le composant de matrice ; et idéalement, comprenant du collagène, éventuellement sous la forme d'une couche de collagène.

3. Dispositif selon la revendication 1, le composé à base de calcium comprenant un composé de phosphate octacalcique biomimétique ; de préférence, fourni sous forme granulaire et ayant une taille de particule qui est optimisée pour la prolifération cellulaire et la différenciation en ostéoblastes, les granules comprenant des granules ayant des tailles de diamètre choisies dans l'une des plages de diamètre suivantes : de 53 à 300 µm, de 300 à 500 µm et de 500 à 1 000 µm.

4. Dispositif selon la revendication 1, l'agent générateur d'os biomimétique (précurseur d'os) comprenant un sel à base de phosphate de calcium.

5. Dispositif selon l'une quelconque des revendications 1 à 4, la phase de génération d'os biomimétique comprenant une phase calcique comprenant un ou plusieurs éléments choisis dans le groupe suivant de composés calciques : phosphate octacalcique (OCP) et/ou phosphate tricalcique (TCP) et/ou hydroxyapatite (HA) et/ou phosphate dicalcique dihydrate (DCPD) et/ou phosphate dicalcique anhydre (DCPA) et/ou phosphate de calcium amorphe (ACP) et/ou carbonate de calcium.

6. Dispositif selon la revendication 1, l'agent générateur d'os biomimétique étant encapsulé dans au moins un composant de matrice.

7. Dispositif selon l'une quelconque des revendications 1 à 5, l'agent générateur d'os biomimétique étant fourni entre le premier composant de matrice et le deuxième composant de matrice et, de préférence, l'agent générateur d'os biomimétique étant fourni sous forme de couche entre le premier composant de matrice et le deuxième composant de matrice.

8. Dispositif selon l'une quelconque des revendications précédentes, la ou chaque matrice ostéoconductrice comprenant un matériau biologique, de préférence un biomatériau poreux, de préférence le biomatériau étant choisi parmi un ou plusieurs des éléments suivants : os trabéculaire d'allogreffe ou de xénogreffe, matrice osseuse déminéralisée (DBM), collagène, hydroxyapatite (HA), acide polylactique ou polyglycolique, verres bioactifs, céramiques à base de calcium, et hydrogel ou le biomatériau étant choisi parmi un ou plusieurs des éléments suivants : collagène et hydrogel.

9. Dispositif selon la revendication 1 - 2, le ou les composants de matrice comprenant des feuilles de collagène et le ou les composants de matrice étant rincés avec de l'oxygène de sorte que des bulles d'air soient piégées entre le premier et le deuxième composant de matrice, par des granules de B-TCP/OCT de taille différente, ou des bulles d'air soient piégées dans les fibres du collagène, pour être utilisées par les cellules selon les besoins.

10. Dispositif selon l'une quelconque des revendications 1 à 5, le précurseur osseux biomimétique comprenant du phosphate octacalcique (OCP) sous forme granulaire, en combinaison avec du phosphate dicalcique dihydrate, avec une taille de particule optimisée pour la prolifération cellulaire.
